# EUROPEAN PATENT APPLICATION

(11) **EP 2 058 002 A1**
(43) Date of publication of application: **13.05.2009**
(21) Application number: 07075946.9
(22) Date of filing: 31.10.2007
(51) Int. Cl.: A61K 39/155, A61K 39/39, A61K 47/48

(54) **Reconstituted respiratory syncytial virus membranes and use as respiratory syncytial virus vaccine**

(71) Applicant: Bestewil Holding B.V., 2333 AL Leiden (NL)
(72) Inventor: Stegmann, Antonius Johannes Hendrikus, 2224 AA Katwijk (NL); Wilschut, Jan Christiaan, 9893 PG Garnwerd (NL); Meijerhof, Tjarko, 9313 JG Groningen (NL)

(57) **Abstract**

The invention relates to reconstituted membranes of Respiratory Syncytial Virus. The invention also relates to a vaccine for Respiratory Syncytial Virus. The invention further relates to methods of production of virosomes comprising reconstituted Respiratory Syncytial virus membranes.

## Description

### Field of the invention

The invention relates to reconstituted membranes of Respiratory Syncytial Virus. The invention also relates to a vaccine for Respiratory Syncytial Virus. The invention further relates to methods of production of virosomes comprising reconstituted Respiratory Syncytial virus membranes.

### Background of the invention

Respiratory Syncytial virus (RS virus) causes severe lower respiratory tract disease in the elderly and infants. An RS virus vaccine is not currently available. Vaccines against membrane-containing (enveloped) viruses mostly consist of whole inactivated or live attenuated virus formulations, or a preparation of their proteins (e.g. split virus vaccines or subunit preparations). Vaccination with killed viruses and protein preparations or subunit vaccines is generally safer than vaccination with live attenuated viruses, because the latter may cause disease or mutate and revert back to wild-type virus. However, attempts to produce RS virus vaccines based on killed virus, or subunit vaccines, have failed, mostly because the RS virus proteins are not very immunogenic. Moreover, attempts to produce live attenuated, or recombinant, virus vaccines consistently resulted in overattenuated virus or vaccines that were not tolerated. But most importantly, RS virus vaccine development has been inhibited by the phenomenon of "enhanced disease": newborn children vaccinated with an alum-adjuvanted, formaldehyde-inactivated virus ("Lot 100") became seriously ill after subsequent natural infection with live RS virus, leading to the death of two of them. An analysis of the immune response evoked by the "Lot 100" vaccine revealed the following problems:
(1) Formaldehyde inactivation selectively reduces the immune response to protective epitopes (those eliciting virus neutralizing antibodies) of the F protein, leading to inadequate levels of neutralizing antibodies (Dudas and Karron, 1998)
(2) In mice, the vaccine alters the balance of Thelper1 (Th1) to Thelper2 (Th2) CD4⁺ cells. Th2 cells are found to predominate, whereas usually in mice Th1 are present in large numbers during unmodified RS virus infection. The Th2 response generates CD4⁺ memory cells, and these inflicted considerable damage onto the sites of infection upon live virus challenge, leading an infiltration of the lungs by eosinophiles and alveolitis), while, in addition, young infants have an inherent bias toward Th2 responses (Dudas and Karron, 1998, Openshaw, 2002)
(3) The vaccine did not, or not sufficiently, prime a cellular immune response leading to the production of interferon γ (IFNγ). In mice, in the absence of an early IFNγ response, a Th2 cytokine profile persists, leading to lung eosinophilia (Openshaw, 2002).

Experiments aimed at reinforcing the immune response to subunit preparations of viruses by physical or chemical means have demonstrated that it is advantageous to combine multiple copies of the viral antigens in particles, that will be taken up efficiently by phagocytic cells. These particles can be Immune-Stimulating Complexes (ISCOMs), proteosome preparations, proteins on microparticle carriers, virosomes, or reconstituted viral membranes. Frequently, chemical substances intended to stimulate the immune system (called adjuvants), are added, which address specific receptors on the phagocytes or the effector cells of the immune system.

The concept of using virosomes for vaccination purposes, particularly for vaccination against influenza, was introduced by Almeida et al.(1975).

After binding to receptor(s) on their host cells, enveloped viruses enter these cells by membrane fusion, delivering the viral genome into the cytoplasm of the host cell. Likewise, virosomes or reconstituted viral membranes enter antigen-presenting cells by membrane fusion, delivering antigens into the cytoplasm of these cells, leading to MHC-I restricted exposure of antigen. Thereby, these particles can induce an immune response typical of infectious virus, without causing a virus infection. Virosomes or reconstituted membranes that are particularly suited for use as vaccines have well-preserved receptor-binding and membrane fusion activities.

Virosomes are lipid bilayers containing viral glycoproteins. Virosomes comprise reconstituted viral membranes. Virosomes may also comprise membranes formed from purified viral proteins and synthetic or natural lipids, or other substances that will form a bilayer. A characteristic feature of virosomes is that they closely mimic the composition, surface architecture and functional activities of the native viral envelope. A particularly important characteristic of said virosomes involves the preservation of the receptor-binding and membrane fusion activity of the native viral envelope, allowing the virosomes to enter the same cells that the virus would be able to enter, and to be presented to the immune system by these same cells. Preservation of receptor-binding and membrane fusion activity is essential for expression of the full immunogenic properties of said virosomes (Arkema 2000; Bungener 2002).

Processes used for the production of virosomes from viruses are known in the art and vary, but always include a step in which the membrane of the virus is disrupted with e.g. a detergent, followed by detergent removal, reconstituting the membrane to form the virosome particle.

The critical step in reconstitution of the membrane, leading to particle formation, is greatly facilitated if the detergents used to solubilize the membrane are easily removed. Current detergent removal protocols that result in reconstitution, such as the one which applicant has earlier developed for influenza (Stegmann et al., 1987), are mostly based on detergents with a low critical micelle concentration (cmc), and such detergents are particularly difficult to remove, in contrast to detergents with a comparatively high cmc, which can be removed by dialysis or ultrafiltration. However, it was found that the the use of latter type of detergent does not generally result in formation of properly reconstituted viral membranes, including the influenza virus membrane, but rather leads predominantly to the formation of empty membranes on the one hand, and protein aggregates on the other. For example, a widely used detergent is the nonionic *n*-octyl-β-D-glucopyranoside, or octylglucoside, which has a high cmc of about 20-25 mM. In our hands, however, attempts to reconstitute influenza membranes from octylglucoside-solubilised virus were unsuccessful in a variety of conditions, and substantially no fusogenic particles were obtained (Stegmann et al., 1987). Therefore, detergents with a high cmc are used in a number of reconstitution protocol, necessitating elaborate protocols for the removal of the detergent. In the process of influenza virus reconstitution by the nonionic detergent octaethyleneglycol-mono-*n*-dodecyl monoether (C12E8) with a low cmc, detergent is removed from the supernatant by a hydrophobic resin (Bio-Beads SM-2), and it was shown (Stegmann et al., 1987 and WO 92/19267) that virus reconstituted in this manner retained its original receptor-binding and membrane fusion activity. Although such virosomes can indeed elicit strong, protective, immune responses (e.g. WO 1988/08718 WO 1992/19267, and WO 2004/110486), removal of detergent from the supernatant by a hydrophobic resin can hardly be scaled up.

Therefore, as an alternative, applicant has shown earlier that influenza virus membranes and Semliki Forest Virus membranes can be solubilized with a short-chain phospholipid such as dicaproylphosphatidylcholine (DCPC), which is well removable by dialysis (WO 2004/071492 and De Jonge et al., 2006). The membrane-fusion activities of the influenza virosomes or reconstituted influenza membranes produced by the DCPC-based method corresponded well to the pH-dependent fusion characteristics of the intact viruses, and to those of virosomes prepared with C12E8, and were found to induce strong immune responses to the major membrane protein of influenza, hemagglutinin (HA).

Influenza virosomes prepared using the C12E8 protocol, carrying a purified RS virus F protein, have been used as vaccines for RS virus. It was found that mice vaccinated intramuscularly with this vaccine, and subsequently vaccinated intranasally with this vaccine plus the heat-labile toxin from *E. coli* as an adjuvant, produced high titer antibodies to RS virus (Cusi et al., 2002). However, upon live virus challenge, virus titers were reduced by only about one log (from 10^{4.4} TCID₅₀ per 100 mg of lung tissue to 10^{3.3}), indicating a lack of virus neutralization. Moreover, with this virosome vaccine, about a 20:1 RS virus-specific serum IgG1 to IgG2a ratio was noted, indicative of a Th2 type immune response. Furthermore, the heat-labile protein of *E. coli* is an unsafe addition to a vaccine. When the heat-labile toxin from *E. coli,* mixed with a virosomal influenza vaccine (EP 0 538 437) was used in an intranasal influenza vaccine, clinical trials indicated that addition of the toxin was absolutely required to induce serum antibody titers equivalent to injected vaccine (Glück et al. 1994). Although addition of the toxin did thus enhance the immunogenicity of this vaccine, it also induced a serious side effect known as Bell's Palsy, a temporary paralysis of facial muscles (Mutsch et al., 2004).

Since the adjuvanting effect of the toxin is due to recognition of the toxin by an antigen-presenting cell, there is no certainty that toxin and viral protein will contact the same cell, and therefore a relatively high concentration of the toxin will be needed in order to ensure activation of every cell, increasing the chance that antigens will be recognized by an activated cell.

Clearly, if virosomes or reconstituted viral membranes are to be used as a vaccine for RS virus, some major improvement in efficacy and safety will be needed. It is well recognized in the art that, in order to develop efficacious and safe RS virus vaccines, vaccines are needed that induce the formation of virus-neutralizing antibodies, lead to a balanced Th1/Th2 response, do not lead to enhanced disease upon a live virus challenge, and induce IFNγ shortly following vaccination and live virus challenge.

### Summary of the invention

The present invention relates to novel means and methods that solve a number of problems and difficulties associated with the development of a safe and efficacious RSV virus vaccine outlined above. The invention relates to a reconstituted RS virus membrane, which may be comprised in a virosome optionally together with an amphiphilic adjuvant, wherein said adjuvant and viral membrane proteins are both present in the same membrane, and associate with the interior of the lipid bilayer membrane through hydrophobic interactions. The RS virosome or reconstituted membrane may be produced by a method involving dissolution of RS virus membranes in a solution containing a short-chain phospholipid, removal of the viral nucleocapsid, and reconstitution of the membrane by dialysis or ultrafiltration of the short-chain phospholipid. The RS virosomes and reconstituted membranes are characterized in that they induce the formation of virus neutralizing antibodies, have a balanced Th1/Th2 response, induce IFNγ upon a live virus challenge, and that they substantially do not induce "enhanced disease".

In particular, the invention relates to a reconstituted viral membrane comprising the membrane proteins and natural lipids of a Respiratory Syncytial virus. The reconstituted viral membrane may further comprise the reconstituted membrane of another enveloped virus, such as of the influenza virus, comprising membrane proteins and natural lipids of the other virus, to form a mixed reconstituted viral membrane.

The invention also relates to a virosome comprising a (mixed) reconstituted viral membrane of the invention. The virosome may further comprise natural or synthetic lipids, not derived from the viral membrane, and/or an amphiphilic adjuvant

The invention further relates to a method of producing a reconstituted viral membrane or a virosome of the invention, comprising the steps of (a) contacting an enveloped virus with a solution containing a short-chain phospholipid allowing solubilization of the viral envelope of said virus; (b) optionally, removing the viral nucleocapsid form the solubilized virus by centrifugation, producing a supernatant containing the solubilized viral membrane; (c) optionally, adding lipid(s) to the solubilized viral membrane; (d) optionally, adding amphiphlic adjuvant(s) to the viral membrane; and (e) further comprising removing short-chain phospholipid from said solution by dialyis or filtration allowing formation of a functionally reconstituted viral envelope. The invention further relates to a method of producing a mixed reconstituted viral membrane.

The invention relates to the use of a (mixed) reconstituted viral membrane or a virosome of the invention, or obtained by a method of the invention, for the preparation of a vaccine and for the prevention, alleviation or treatment of a RSV infection.

The invention also relates to a pharmaceutical composition comprising a reconstituted viral membrane or a virosome of the invention, or obtained by a method of the invention, and optionally a pharmaceutically acceptable carrier. The pharmaceutical composition is suitable for intranasal, oral or parenteral administration.

The invention further relates to a vaccine comprising a (mixed) reconstituted viral membrane or a virosome of the invention, or obtained by a method of the invention, and optionally a pharmaceutically acceptable carrier or diluent and/or an adjuvant. The vaccine is suitable for intranasal, oral or parenteral administration.

The invention further relates to a method for the vaccination of a subject comprising the step of administering a reconstituted viral membrane or a virosome according to the invention or obtained by a method according to the invention, for the prevention, alleviation or treatment of a RSV infection.

### Description of the invention

Inventors surprisingly found that reconstituted RS virus membranes, or virosomes comprising these (RS virosomes), are a safe and effective vaccine against RS virus.

Indeed, inventors demonstrate that (a) RS virus can be effectively solubilized and reconstituted with a short-chain phospholipid, such as DCPC, by dialysis, into a reconstituted RS membrane or virosome; (b) RS virus and influenza virus can be solubilized with such short-chain phospholipids, and reconstituted into a mixed reconstituted RS membrane or virosome together; (c) both RS virus and RS virus/influenza virosomes can be produced to comprise an amphiphilic adjuvant, such as a lipopeptide. All the above preparations induce immunity to RS virus. However, the immunogenicity and safety of the reconstituted RS virus membranes or virosomes, as opposed to RS virus/influenza, are superior, and a further improvement in immunogenicity can be obtained by the inclusion of the adjuvant into the membrane. RS virus virosomes with a lipopeptide adjuvant were found to induce high titers of virus-neutralizing antibodies, a balanced Th1/Th2 profile after infectious RS virus challenge, sterilizing immunity upon virus challenge, the secretion of IFN-γ from spleen cells after challenge, both in an MHC-I and MHC-II restricted fashion, without the lung pathology that is characteristics of "enhanced disease".

It was found that reconstituted RS membranes, and virosomes comprising these, in contrast to influenza virosomes comprising a purified RS virus F protein, elicit a strong humoral and cellular immune response, capable of neutralizing a live virus challenge in mice and cotton rats, without causing "enhanced disease". The immune response could be further enhanced by the presence of an amphiphilic adjuvant in the reconstituted viral membrane or virosome.

The present invention provides a reconstituted viral membrane, comprising membrane proteins and natural lipids of a Respiratory Syncytial virus. Reconstituted viral membranes of the invention comprise all or some of the natural proteins and lipids of the membrane of the virus they were produced from. Naturally occurring membrane proteins of RS virus include F, G, and SH.

The term "natural lipids of a virus" is herein understood to mean those lipids that are present in the membrane of a virus grown on cells, preferably mammalian. The natural lipids of a virus are thus preferably obtained or isolated from virus particles thus grown, as opposed to synthetic lipids, and generally comprise phosphatidylcholine, phosphatidylethanolamine, sphingomyelin, cholesterol and phosphatidylserine. The RS virus G and/or SH protein have been shown to form a complex with the F protein in a natural RS virus membrane (Feldman et al., 2001).

In general, such reconstituted membranes and/or virosomes are produced by dissolving the viral membrane through the action of a detergent or other substance, followed by removing viral genetic material, generally comprised in a nucleocapsid, through centrifugation, leaving a supernatant that comprises all or part of the viral membrane material, dissolved in the substance. The detergent or other dissolving substance may preferentially dissolve some of the membrane components such as proteins and lipids, but not others. Then, the membrane can be reformed, by removal of the detergent or other substance, leading to the formation of reconstituted viral membranes.

After solubilization of viral membranes as described above, but prior to removal of the detergent or other substance, other material, such as proteins, lipids, nucleic acids or drugs can be added to the solubilized membranes.

The invention further provides virosomes, comprising the reconstituted membranes of RS virus. Virosomes as meant herein are produced from viral envelopes, optionally comprising an amphiphilic adjuvant, and also comprising material not derived from the viral envelope in their membrane or lumen. "Reconstituted viral membranes" as meant herein comprise natural lipids and proteins of an enveloped virus, optionally comprising an amphiphilic adjuvant. Virosomes and reconstituted viral membranes have membrane fusion activity, as evidenced by MHC-I restricted immune responses after vaccination of animals with the virosomes. In the art, "virosomes" and "reconstituted viral membranes" are often used interchangeably.

Virosomes of the invention may further comprise purified lipids from other sources, e.g. synthetic lipids, in addition to the natural lipids. A lipid composition for the provision of the virosomes with fusion activity is thus preferably a composition exclusively composed of natural lipids of a virus, the lipids present in natural host cells for the virus, compositions composed of natural lipids of a virus supplemented with lipids from other sources, as well as compositions composed of lipids from various sources, which mimic the lipid composition of a natural viral membrane.

Prior to the removal of the detergent or other solubilizing substance from solubilized viral membranes, optionally supplemented with lipids or other materials as mentioned above, other hydrophobic, amphiphilic material, or water-soluble material can be added for inclusion in virosomes, and will end up in the virosome lumen, or be included in the membrane if sufficiently hydrophobic. Lipids, and amphiphilic adjuvants are examples of substances that are sufficiently hydrophobic for their inclusion in the membrane, while proteins such as ovalbumin are enclosed within their lumen. Characteristic of both the virosome and the reconstituted viral membrane is the lipid bilayer membrane, which comprises all or some of the viral lipids, and all or some of the viral membrane proteins.

Reconstituted viral membranes and/or virosomes can be formed from all enveloped viruses. Influenza virus is a classical example of an enveloped virus. The first step in infection of cells by influenza virus is uptake of intact viral particles by receptor-mediated endocytosis. Inside the endosomal compartment the conditions are mildly acidic (pH 5 - 6). Triggered by the endosomal pH, the viral spike protein hemagglutinin (HA) undergoes a conformational change which results in triggering of viral membrane fusion activity. Subsequent fusion of the viral membrane with that of the endosome results in cytoplasmic penetration of the viral genome, and the cell can be considered infected.

While influenza enters cells through receptor-mediated endocytosis and pH-triggered fusion from within an acidic endosome, other viruses induce membrane fusion in a pH-independent fashion. Non-limiting examples of such viruses are: Retroviridae such as Human Immunodeficiency virus; paramyxoviridae such as parainfluenza viruses, measles, mumps, RS virus and human metapneumovirus. Although it is sometimes thought that pH-independent fusion implies direct fusion of the envelope of these viruses with the plasma membrane of the cells, this is not actually known for many of these viruses. Fusion may also occur from within an acidic endosome, like that seen with influenza virus, but is not triggered by low pH, unlike that of influenza virus. In fact, there are many structural and functional similarities between the membrane fusion proteins of the paramyxoviruses and influenza HA (Lamb et al., 2006). Both are class I membrane fusion proteins, homotrimers of proteins composed of two disulfide-linked subunits each, one of which is anchored in the viral membrane, and carries a hydrophobic sequence at the membrane-distal end that is known as a "fusion peptide". Sequences adjacent to the fusion peptide reveal a heptad repeat pattern of hydrophobic amino acids. In HA-induced fusion, a major conformational rearrangement induced by low pH exposes the fusion peptide, which is buried in the HA protein moiety at neutral pH. The conformational change finally results in the formation of a coiled-coil structure involving the heptad repeat region. The fusion proteins of the paramyxoviruses undergo a similar conformational change, exposing the fusion peptide, and leading to coiled-coil formation in the heptad region to drive fusion, but the precise trigger for this is not low pH and is not always known.Reconstituted RS membranes and virosomes of the present invention closely mimick the composition, surface architecture and functional activities of the native viral envelope. Reconstituted RS membranes and virosomes that are particularly active in inducing an immune response were found to have maintained the proper functions of the envelope proteins of the native virus, such as receptor-binding, membrane fusion and other activities. Thus, preservation of receptor-binding and membrane fusion activity is essential for expression of full immunogenic properties of said virosomes.

The present invention also provides a mixed reconstituted viral membrane, comprising the reconstituted viral membrane of a Respiratory Syncytial virus, as described above, and further a reconstituted membrane of another enveloped virus, comprising membrane proteins and natural lipids. Preferentially, the mixed reconstituted viral membranes are prepared by dissolving RS and a different enveloped virus by means of the same, or a compatible, detergent, or other dissolving substance, mixing the membranes, and removing the detergent or other agent from the solution, leading to the formation of a reconstituted or virosomal membrane comprising components of both membranes, for example comprising the reconstituted membrane, or the fusion proteins (HA) of influenza virus. Compatible solubilizing substances are substances that can be removed in a similar fashion, for example dialysis. "Mixed" reconstituted membranes and/or virosomes comprise the membrane fusion properties of either or both of the constituent viruses, allowing for example the introduction of membrane material of a virus that normally fuses with the plasma membrane of a cell at neutral pH, through the endosome at low pH. Preferably, the lipid composition is compatible with fusion at the optimal pH of fusion. "Mixed" reconstituted membranes may comprise antigens from both viruses, eliciting an immune response in a synergistic fashion, or modulating the immune response otherwise. In a preferred embodiment the mixed reconstituted viral membrane comprises reconstituted viral membranes of Respiratory Syncytial virus and influenza virus.

The invention further provides for reconstituted RS virus membranes, RS virosomes, mixed membranes and/or virosomes comprising these, comprising an amphiphilic adjuvant. In the reconstituted viral membrane, preferably, the lipid bilayer has a lipid composition that is compatible with fusion, as induced by the fusion protein(s), of the virosomal membrane with a host cell of a natural host of the virus. Preferably, the fusion protein(s), the amphiphilic adjuvant and preferably also the further viral membrane proteins interact with the hydrophobic interior of the lipid bilayer, i.e. are associated with, integrated into, and/or embedded in the bilayer of the viral membrane through hydrophobic interactions with the lipids of the bilayer and/or each other. Further preferred is that the fusion protein(s) and the amphiphilic adjuvant are not covalently linked. Preferably, the amphiphilic adjuvant and the further viral antigen are also not covalently linked. In the preferred viral membrane of the invention, an amphiphilic adjuvant, a viral fusion protein and optional further viral proteins interact primarily through hydrophobic interactions, wherein the hydrophobic part of the amphiphilic adjuvant preferably forms an integral part of a lipid bilayer membrane, which bilayer further comprises the fusion protein(s), other viral proteins and lipids. A preferred reconstituted viral membrane is a vesicle approximately the size of the virus it is derived from.

A "fusion protein of a virus" is herein understood to mean an integral membrane protein of a virus, usually an enveloped virus that, if expressed on the surface of a suitable cell, can induce fusion of the cell, at an appropriate pH, with cells that are a natural host for the virus. Examples of viral fusion proteins for incorporation into the reconstituted viral membrane are the influenza virus hemagglutinin (HA) protein, and the F proteins of RS virus. The capability of the reconstituted viral membranes of the invention to fuse with a host cell is thus dependent on the presence of an appropriate viral fusion protein. However, this capability is further dependent of the lipid composition of the bilayer of the reconstituted viral membrane, as virosomes composed of synthetic lipids and viral fusion proteins have been described in the art that as being incapable of fusion. The lipid composition of the reconstituted viral membranes is thus preferably chosen such that the membranes are capable of fusion with appropriate host cells at an appropriate pH. One preferred lipid composition that provides the reconstituted viral membranes with fusion activity is a lipid composition that comprises or closely mimicks natural lipids of a virus.

The invention also provides a method of producing a reconstituted viral membrane or virosome according to the present invention. The method according to the invention comprises the steps of: (a) contacting an enveloped virus with a solution containing a short-chain phospholipid allowing solubilization of the viral envelope of said virus; (b) optionally, removing the nucleocapsid of the solubilized virus by centrifugation; (c) optionally, adding an amphiphilic adjuvant to the solubilized viral envelope; (d) optionally, adding lipid(s) to the solubilized viral envelope; and (e) removing the short-chain phospholipid from said solution by dialysis or filtration allowing formation of a reconstituted viral envelope or virosome.

The invention further provides a method of producing a mixed reconstituted viral membrane according to the invention or a virosome comprising a mixed reconstituted viral membrane according to the invention, comprising the steps of: (a) contacting a first enveloped virus with a solution containing a short-chain phospholipid allowing solubilization of the viral envelope of said virus; (b) contacting a second enveloped virus with a solution containing a short-chain phospholipid allowing solubilization of the viral envelope of said virus; (c) optionally, removing the nucleocapsid from both viruses from the solubilized virus by centrifugation; (d) mixing the solubilized membranes of both viruses; (e) optionally, adding an amphiphilic adjuvant to the mixture; (f) optionally, adding lipid(s) to the mixture; and (g) removal of the short-chain phospholipid from said mixture by dialysis or filtration allowing formation of a mixed reconstituted viral envelope containing lipids and proteins of both viruses.

The invention further provides for reconstituted RS viral membranes, RS virosomes, mixed reconstituted viral membranes and mixed virosomes, produced from virus by solubilization of the viral membrane with a short-chain phospholipid. In a further preferred embodiment, the method further comprises removal of viral nucleocapsid from the virus solubilized by the short-chain phospholipid, e.g. by centrifugation. Said short-chain phospholipid is preferably removed by dialysis or (ultra)filtration. Suitable short-chain phospholipids have acyl chains with between one and eight carbon atoms, and/or a cmc of larger than 0.1 mM, and can be phosphatic acid, or have a variety of headgroups linked to the phospholipid phosphate such as choline, ethanolamine or serine. It is preferred that said phospholipid is a short-chain phosphatidylcholine. Especially suitable phosphatidylcholines are listed in Table 1.

**Table 1: Preferred phosphatidylcholines of the invention**

| **Phosphatidylcholine** | **CMC** |
|---|---|
| 1,2-dipentoyl-*sn*-phosphatidylcholine (C5) | 90 mM |
| 1,2-dicaproyl-*sn*-phosphatidylcholine (C6) | 14 mM |
| 1,2-diheptanoyl-*sn*-phosphatidylcholine (C7) | 1.4 mM |
| 1,2-dicapryloyl-*sn*-phosphatidylcholine (C8) | 0,27 mM |

Preferred examples of short-chain phospholipids are 1,2-diheptanoyl-*sn-*phosphatidylcholine (DHPC) or 1,2-dicaproyl-*sn*-phosphatidylcholine (DCPC), with seven or six carbon atoms in the acyl chain, respectively. The cmc of such molecules increases as the length of the fatty-acyl chains decreases (Tausk et al., 1974), as shown in Table 1.

"Adjuvants" are herein intended to include any substance or compound that, when used, in combination with an antigen, to immunise a human or an animal, stimulates or modulates the immune system, thereby provoking, enhancing or facilitating the immune response against the antigen, preferably without generating a specific immune response to the adjuvant itself. Preferred adjuvants enhance the immune response against a given antigen by at least a factor of 1.5, 2, 2.5, 5, 10 or 20, as compared to the immune response generated against the antigen under the same conditions but in the absence of the adjuvant. Tests for determining the statistical average enhancement of the immune response against a given antigen as produced by an adjuvant in a group of animals or humans over a corresponding control group are available in the art. The adjuvants to be incorporated in the functionally reconstituted viral membranes of the invention are preferably amphiphilic adjuvants.

The term "amphiphilic adjuvant" is intended to describe any adjuvant, including compounds like lipopeptides and glycolipids, having hydrophobic membrane embedded and environment-oriented polar (head group) moieties and which, preferably by itself, can associate with, or more preferably integrate into lipid bilayer vesicles or micelles in water. The term also includes any amphiphilic adjuvant that is stably incorporated into lipid bilayers (comprising the natural lipids of a virus) with its hydrophobic moiety in contact with the interior, hydrophobic region of the bilayer membrane, and its polar head group moiety oriented toward the exterior, polar surface of the membrane. However, more hydrophobic adjuvants having a less pronounced amphiphilicity, i.e. having no or only weakly polar head group moieties, but which can associate with, or integrate into lipid bilayer vesicles, are explicitly not excluded from the invention. The "amphiphilic adjuvants" with adjuvant activity as used herein, thus include naturally occurring or (partly) synthetic adjuvants that are capable of forming a reconstituted viral membrane together with one or more antigens of interest and natural lipids of a virus in an aqueous environment under conditions that allow the formation of a reconstituted viral membrane.

In a preferred embodiment, the amphiphilic adjuvant present in the reconstituted viral membrane is pharmaceutically acceptable for use in humans, in contrast to e.g. Quil A™ or some other saponins, which are amphiphiles with adjuvant activity that have been tested in certain settings in the art. The amphiphilic adjuvants of the invention are preferably not covalently linked to the antigens but are present together in the lipid bilayer of the reconstituted membrane. The fact that the antigen and the adjuvant are not covalently linked assures that processing of the antigen and presentation of its epitopes to the immune system is essentially identical to that of the natural protein alone, ensuring good recognition of the protein present on the natural pathogen. On the other hand, the hydrophobic interaction of the antigen and the adjuvant with the lipid bilayer (and each other) allows for a distribution of the adjuvant and antigen over the reconstituted viral membranes in a preparation whereby the majority of the membrane vesicles in a preparation comprise both the antigen and adjuvant in a single vesicle, more preferably at least 40, 60, 80, 90, 95 or 95% of the vesicles comprise both the antigen and adjuvant. The combination of antigen and adjuvant in a single membrane or vesicle allows delivery of the antigen to the antigen presenting cell that is activated by the adjuvant, thereby increasing the immunogenicity of the reconstituted viral membranes.

In a preferred embodiment of the invention said amphiphilic adjuvant is recognized by a Toll-like-receptor (TLR) present on antigen presenting cells. Various compounds recognized by TLRs are known in the art and include e.g. lipopeptides, lipopolysaccharides, peptidoglycans, lipoteichoic acids, lipoproteins (from mycoplasma, mycobacteria or spirochetes), double-stranded RNA (poly I:C), unmethylated DNA, lipoarabinomannan, flagellin, CpG-containing DNA, and imidazoquinolines. Not all TLR-recognised compounds are suitable as adjuvants, as e.g. the toxicity of wild-type Gram-negative bacterial lipopolysaccharides is too high for them to be used as adjuvants, i.e. they are not pharmaceutically acceptable for use in humans. Other TLR-recognised compounds may however be used as adjuvants. Such TLR-recognized adjuvants may be amphiphilic adjuvants by themselves, or alternatively they may be modified into an amphiphilic adjuvant, e.g. by coupling hydrophobic compounds (see below) to a polar TLR ligand. Alternatively, the amphiphilic adjuvants may target other receptors.

A preferred amphiphilic adjuvant is a lipopeptide, which may be produced synthetically or semi-synthetically. A preferred lipopeptide for use as amphiphilic adjuvant has adjuvant activity and is pharmaceutically acceptable for use in humans. A lipopeptide of the invention is a molecule that will usually consist of one or more (oligo)peptides covalently coupled to one or more hydrophobic compounds selected from fatty acids, lipids, ceramides, plasmalogens, alkyl or alkene chains, or sterols. Generally, lipopeptides for use in the present invention preferably comprise 3, 4, 5, 6, 7, or 8, amino acids, preferably the peptides comprise 40 - 70% amino acids that are positively charged, of which lysine and arginine are preferred, and preferably the peptides comprises one or more serines and/or cysteines. Especially preferred lipopeptides of the invention are listed in Table 2.

**Table 2: Lipopeptides particularly suitable for making reconstituted viral membranes according to the invention.**

| **Lipopeptide** |
|---|
| *N*-palmitoyl-S-2,3(bispalmitoyloxy)-propyl-cysteinyl-seryl-serine |
| S-2,3(bispalmitoyloxy)-propyl-cysteinyl-seryl-serine |
| *N*-palmitoyl-S-2,3(bispalmitoyloxy)-propyl-cysteinyl-seryl-(lysil)₃-lysine |
| S-2,3(bispalmitoyloxy)-propyl-cysteinyl-seryl-(lysil)₃-lysine |
| *N*-palmitoyl-S-2,3(bisoleoyloxy)-propyl-cysteinyl-seryl(lysil)₃-lysine |
| S-2,3(bisoleoyloxy)-propyl-cysteinyl-seryl-(lysil)₃-lysine |
| *N*-palmitoyl-S-2,3(bismyristoyloxy)-propyl-cysteinyl-seryl-(lysil)₃-lysine |
| S-2,3(bismyristoyloxy)-propyl-cysteinyl-seryl-(lysil)₃-lysine |
| *N*-palmitoyl-S-3(palmitoyloxy)-propyl-cysteinyl-seryl-(lysil)₃-lysine |
| *N*-palmitoyl-S-2,3 hydroxy-propyl-cysteinyl-seryl-(lysil)₃-lysine |
| *N*-palmitoyl-S-2,3(bispalmitoyloxy)-propyl-cysteinyl-seryl-(prolyl)₃-proline |
| *N*-palmitoyl-S-2,3(bispalmitoyloxy)-propyl-cysteinyl-seryl-(glutaminyl)₃-glutaminic acid |

In another embodiment of the invention said amphiphilic adjuvant is a glycolipid. A preferred glycolipid for use as amphiphilic adjuvant has immunological adjuvant activity and is pharmaceutically acceptable for use in humans. Glycolipids are lipids covalently coupled to one or more sugars. In a highly preferred embodiment the invention provides reconstituted viral membranes according to the invention, in which the glycolipid is a α-galactosylceramide or a phosphatidyl inositol mannoside. The terms "an α-galactosylceramide" and "a phosphatidyl inositol mannoside" are intended to include any derivative of either one. Derivatives of these molecules having adjuvant activity and that are useful in the context of the present invention are e.g. described in US 5,936,076 and in US 4,542,212, respectively. Other suitable glycolipid adjuvants for use in the invention include e.g. modified forms of endotoxic lipopolysaccharides (LPS) of Gram-negative bacteria having reduced toxicity of the Lipid A portion of the LPS but retaining (part of) the adjuvant activity, as may be obtained from genetically modified Gram-negative pathogens and as reviewed in WO02/09746.

A modified LPS for use as amphiphilic adjuvant in the invention preferably has a modified Lipid A moiety with reduced toxicity.

In another aspect of the present invention, the amphiphilic adjuvant present in the virosome according to the invention, is a peptide, preferably an amphiphilic peptide. A preferred peptide for use as amphiphilic adjuvant has immunological adjuvant activity and is pharmaceutically acceptable for use in humans. Peptides, in particular polar peptides, with adjuvant activity may be rendered into amphiphilic adjuvants by (covalently) linking them to a suitable hydrophobic compound (see above). Alternatively, amphiphilic peptides may comprise a hydrophobic stretch of amino acids such as a transmembrane sequence as described below. A preferred peptide comprises a sequence from the Notch ligand Jagged-1 (see Weijzen et al., 2002; Genbank accession no. AAC 52020) or a sequence from the *Staphylococcus aureus* protein A. Peptides having sequences from Jagged-1 or protein A are preferably covalently coupled to a suitable hydrophobic compound (see above) and/or comprise a transmembrane sequence (see below). The (polar) part of the Jagged-1 or protein

Hydrophobic interactions result from non-covalent, non-electrostatic attraction forces between hydrophobic substances that are present in an aqueous environment.

The reconstituted viral membranes of the invention are preferably suitable for both parenteral and mucosal (e.g. intranasal or oral) administration.

In a further aspect the present invention provides a pharmaceutical composition comprising a reconstituted viral membrane, a mixed reconstituted viral membrane or a virosome according to the invention, or obtainable by a method according to the invention, and optionally a pharmaceutically acceptable carrier.

In one embodiment the present invention provides a pharmaceutical preparation comprising as active ingredient a reconstituted viral membrane according to the invention, and a pharmaceutically acceptable carrier. Pharmaceutically acceptable stabilizing agents, osmotic agents, buffering agents, dispersing agents, and the like may also be incorporated into the pharmaceutical composition. The preferred form depends on the intended mode of administration and therapeutic application. The pharmaceutical carrier can be any compatible, non-toxic substance suitable to deliver the reconstituted viral membranes to the patient. Pharmaceutically acceptable carriers for intranasal delivery are exemplified by water, buffered saline solutions, glycerin, polysorbate 20, cremophor EL, and an aqueous mixture of caprylic/capric glyceride, and may be buffered to provide a neutral pH environment. Pharmaceutically acceptable carriers for parenteral delivery are exemplified by sterile buffered 0.9% NaCl or 5% glucose optionally supplemented with a 20% albumin. Preparations for parental administration must be sterile.

In one embodiment the pharmaceutical composition according to the invention is suitable for intranasal, oral or parenteral administration.

The parental route for administration of the polypeptide or antibody is in accord with known methods, e.g. injection or infusion by intravenous, intraperitoneal, intramuscular, intraarterial or intralesional routes. The reconstituted viral membranes are preferably administered by bolus injection. A typical pharmaceutical composition for intramuscular injection would be made up to comprise, for example, 1 - 10 ml of phosphate buffered saline and 1 to 100 µg, preferably 5-45 µg (of antigen protein) of the reconstituted viral membranes of the present invention. For oral administration, the active ingredient can be administered in liquid dosage forms, such as elixirs, syrups, and suspensions. Liquid dosage forms for oral administration can comprise coloring and flavoring to increase patient acceptance. Methods for preparing parenterally, orally or intranasally administrable compositions are well known in the art and described in more detail in various sources, including, for example, Remington's Pharmaceutical Science (1980) (incorporated by reference in its entirety for all purposes).

In another embodiment the invention provides a method further comprising addition of a molecule that is not derived from said virus to said reconstituted viral membrane to produce a virosome. Such a molecule may be delivered to the cell that takes up the virosome and may for example be a nucleic acid, a lipid or a protein, preferably derived from a pathogen, such as a virus, a bacterium or a parasite, or a tumor-specific molecule. When said molecule is of an amphiphilic nature, it will in general be presented within the context of the membrane of the virosome.

The present invention also provides for the use of a reconstituted viral membrane, a mixed reconstituted viral membrane or a virosome according to the invention or obtainable by a method according to the invention, for the preparation of a vaccine.

The present invention also provides for the use of a reconstituted viral membrane, a mixed reconstituted viral membrane or a virosome according to the invention or obtainable by a method according to the invention, for the preparation of a vaccine for the prevention, alleviation or treatment of a RSV infection.

The present invention still further provides a vaccine comprising a reconstituted viral membrane, a mixed reconstituted viral membrane or a virosome according to the invention or obtainable by a method according to the invention, and optionally a pharmaceutically acceptable carrier or diluent. In one embodiment the vaccine according to the invention is suitable for intranasal, intravaginal, oral or parenteral administration.

A pharmaceutical composition comprising a reconstituted viral membrane according to the invention can also be used as delivery vehicle for nucleic acid or proteins.

In a further aspect, the invention relates to a method for vaccination against RS virus by administration of a therapeutically or prophylactically effective amount of (a pharmaceutical composition comprising) reconstituted viral membranes of the invention to a subject in need of prophylaxis or therapy. As such, the present invention provides a method for the vaccination of a subject comprising the step of administering a reconstituted viral membrane, a mixed reconstituted viral membrane or a virosome according to the present invention or obtainable by a method of the invention.

The present invention further provides a method for the vaccination of a subject for the prevention, alleviation or treatment of a RSV infection comprising the step of administering a reconstituted viral membrane, a mixed reconstituted viral membrane or a virosome according to the present invention or obtainable by a method of the invention.

In the following examples inventors demonstrate that RS virus can be effectively solubilized and reconstituted with a short-chain phospholipid, such as DCPC, by dialysis. Inventors also demonstrate that RS virus and influenza virus can be solubilized with such short-chain phospholipids, and reconstituted into a mixed membrane together. Both RS virus and RS/influenza virosomes can be made to comprise an amphiphilic adjuvant, such as a lipopeptide, by the method. All of these preparations induce immunity to RS virus. However, the immunogenicity and safety of the reconstituted RS virus, as opposed to RS/influenza, are superior, and a further improvement in immunogenicity can be obtained by the inclusion of the adjuvant into the membrane. RS virosomes with the a lipopeptide adjuvant were found to induce high titers of virus-neutralizing antibodies, a balanced Th1/Th2 profile after infectious RS virus challenge, sterilizing immunity upon virus challenge, the secretion of IFN-γ from spleen cells after challenge, both in an MHC-I and MHC-II restricted fashion, without the lung pathology that is characteristic of "enhanced disease".

The examples and figures illustrate, but are not intended to limit, the invention:

### Description of the figures

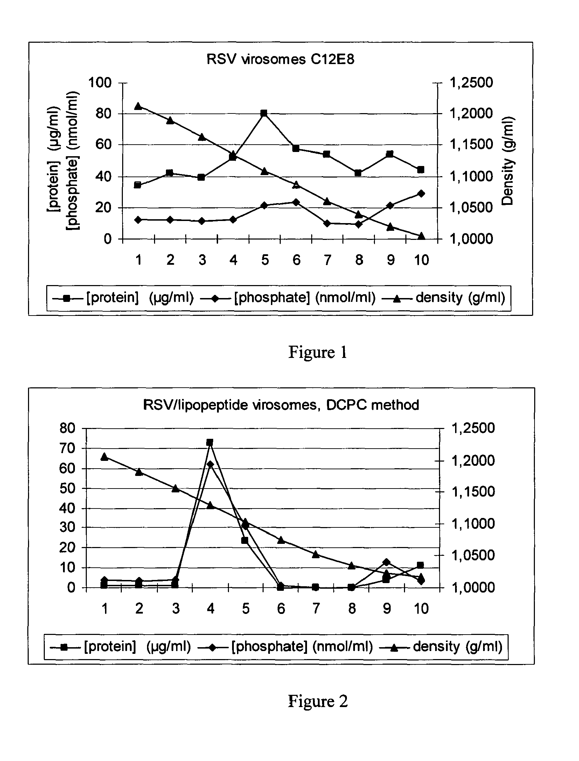

### Figure 1 Reconstitution of the membrane of RS virus, strain A2, by the C12E8-based protocol

Products of the procedure were separated by equilibrium density gradient centrifugation, and fractions were then assayed for (phospholipid) phosphate (diamonds), protein (squares), and density (triangles). Protein and lipid were found to be scattered throughout the gradient, indicating no virosomes have formed.

### Figure 2 Reconstitution of the membrane of RS virus, strain A2, also comprising a lipopeptide adjuvant by the DCPC-based protocol

Products of the procedure were separated by equilibrium density gradient centrifugation, and fractions were then assayed for (phospholipid) phosphate, (diamonds), protein (squares), and density (triangles). Protein and lipid were predominantly found in a single peak at a density of around 1.12 g/ml, indicating virosomes were formed. A small fraction of the material appeared not to be incorporated in virosomes, but remained present near the top of the gradient.

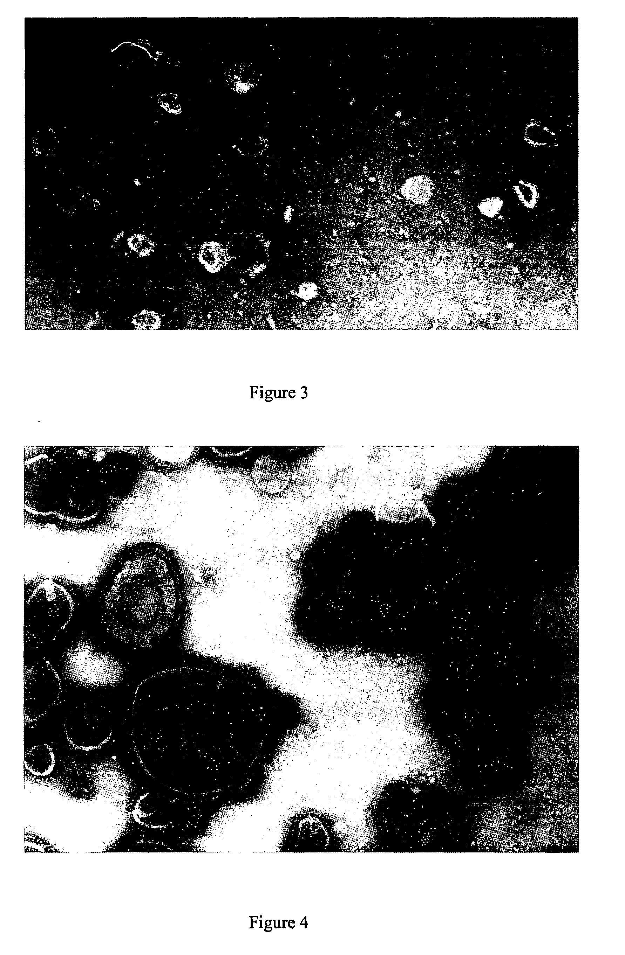

### Figure 3 Electron microcopy of RS virus, strain A2 virosomes comprising a lipopeptide adjuvant, produced by the DCPC-based protocol

Virosomes were deposited on freshly glow-discharged carbon coated grids. After 1 minute, the grids were blotted, stained with 1% uranyl acetate in water, and air-dried. The virosomes are about 100 nm in diameter on average. The RS virus membrane proteins can be seen as a fuzzy white layer surrounding the virosomes, and as small dots atop the virosomes.

### Figure 4 Electron microcopy of RS virus/influenza mixed reconstituted viral membranes produced by the DCPC-based protocol

Virosomes were deposited on freshly glow-discharged carbon coated grids. After 1 minute, the grids were blotted, stained with 1% uranyl acetate in water, and air-dried. The virosomes are about 200 nm in diameter on average. The influenza membrane proteins (predominantly HA) can clearly be seen to project from the membrane.

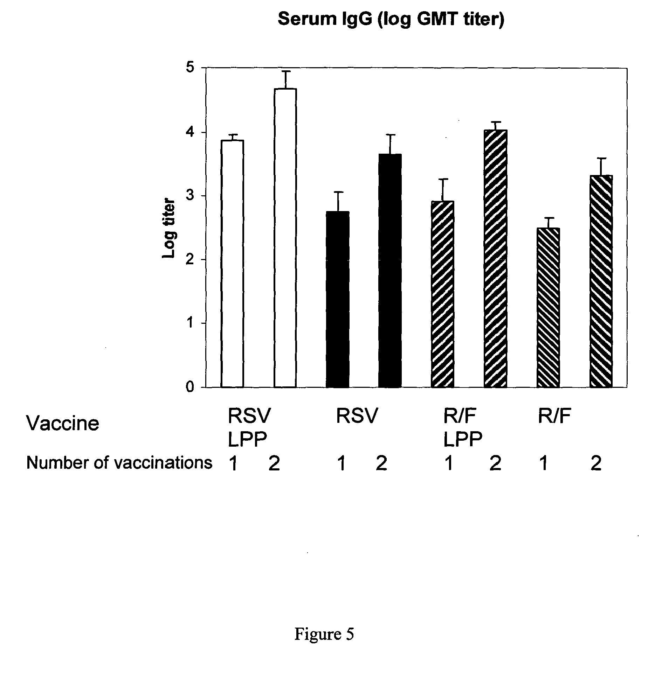

### Figure 5 RS virus-specific serum IgG antibodies induced by vaccination with virosomes made according to the DCPC protocol.

Virosomes were produced from mixed influenza/RS virus (R/F) membrane material, or RS virus membrane material alone (RSV), with or without a lipopeptide adjuvant (LPP), and injected intramuscularly once or twice, with an interval of two weeks between vaccinations into 8-12 week old Balb/C mice. Six mice were used per virosome preparation. Serum was collected two weeks after the injection and assayed for RS virus-specific IgG using ELISA plates coated with virus. Sera collected at the day of the first injection did not have any RS virus specific antibodies. Results are presented as geometric mean titers on a logarithmic scale + one standard deviation.

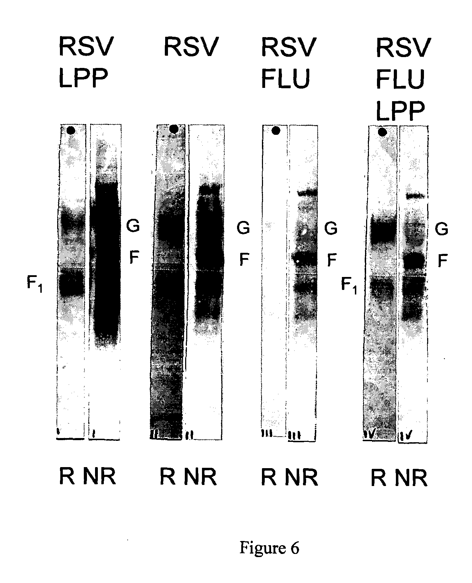

### Figure 6 The antibodies produced by vaccination with RS virosomes, made according to the DCPC method recognize the RS virus F and G proteins.

Western blot of RS virus after SDS-PAGE separation of total viral proteins as know in the art, developed with mouse sera obtained as described in Figure 5 R refers to a gel run under reducing conditions, NR to non-reducing conditions.

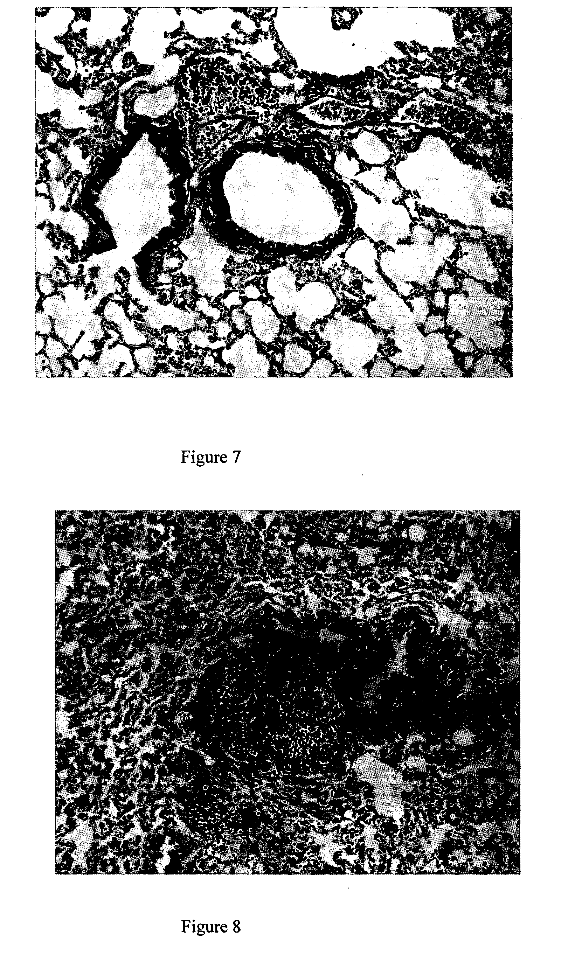

### Figure 7 Micrograph of Giemsa-stained formaldehyde-fixed lung slice from Balb/C mice vaccinated with RS virosomes comprising lipopeptide and challenged with live virus.

8-12 week old Balb/C mice were vaccinated twice at weekly intervals with lipopeptide-comprising virosomes made from the membranes of RS virus strain A2 virus. Two weeks after the last injection, they were challenged with live RS virus A2 virus. Four days after the challenge, the lungs were harvested. A lymphocyte-containing infiltrate can be seen at one side of a blood vessel top left to the middle. The size and frequency of such infiltrates shown in this picture are representative for what found in multiple groups of six mice. 7 days after challenge, these infiltrates vanish (not shown).

### Figure 8 Micrograph of Giemsa-stained formaldehyde-fixed lung slice from Balb/C mice vaccinated with RS virus/influenza virosomes comprising lipopeptide and challenged with live virus.

8-12 week old Balb/C mice were vaccinated twice at weekly intervals with lipopeptide-comprising virosomes made from the membranes of RS virus strain A2 virus mixed with membrane material form influenza strain A/Panama/2007/99. Two weeks after the last injection, the mice were challenged with live RS virus A2 virus. Four days after the challenge, the lungs were harvested and processed for microscopy. A very large lymphocyte-containing infiltrate can be seen surrounding (and apparently, occluding) two blood vessels in the middle of the micrograph. Infiltrates of this size were regularly found in the lungs of these mice.

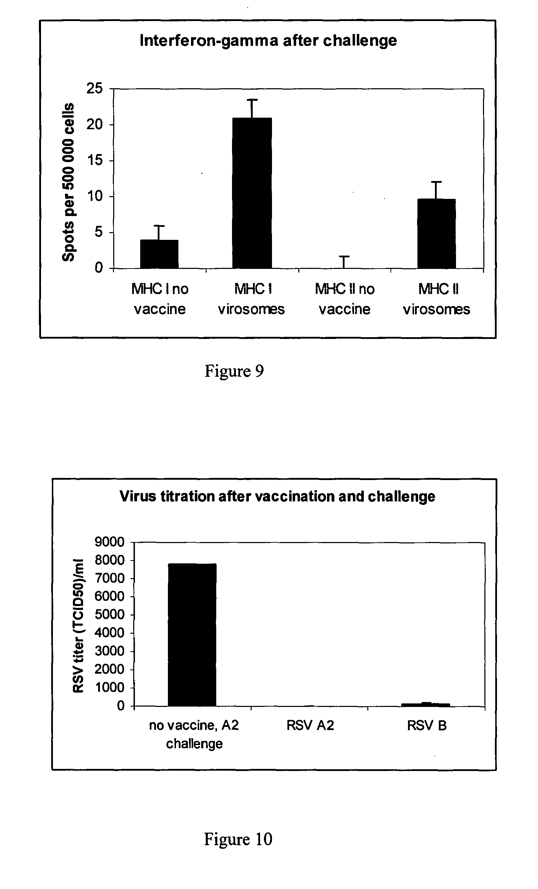

### Figure 9 Induction of IFNγ after challenge

Mice were vaccinated with RS virus/lipopeptide virosomes as described in Figure 7 and challenged with live virus 14 days after the last vaccination. 4 days later the mice were euthanized, and IFNγ secreted by spleen cells was measured by ELISPOT assay after stimulation with gamma-irradiated RS virus infected syngeneic cells ("MHC-I") or inactivated virus ("MCH II"). Control mice were challenged, but not vaccinated.

### Figure 10 Virus titers after vaccination and challenge

Mice were vaccinated with RS virus/lipopeptide virosomes made from the A2 or B strain respectively, were vaccinated as described in Figure 7 and challenged with live virus of the respective strain 14 days after the last vaccination. 4 days later the mice were euthanized, and virus titers in lung tissue determined as indicated. Control mice were not vaccinated, and challenged with A2 virus.

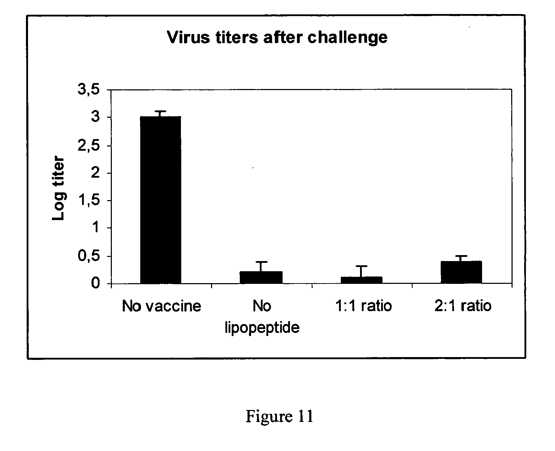

### Figure 11 Virus titers after vaccination with virosomes comprising different concentrations of lipopeptide, and challenge

Mice were vaccinated with RS virus A2/lipopeptide virosomes, were vaccinated as described in Figure 7 and challenged with live virus 14 days after the last vaccination. 4 days later the mice were euthanized, and virus titers in lung tissue determined as indicated. Control mice were not vaccinated, and challenged with virus.

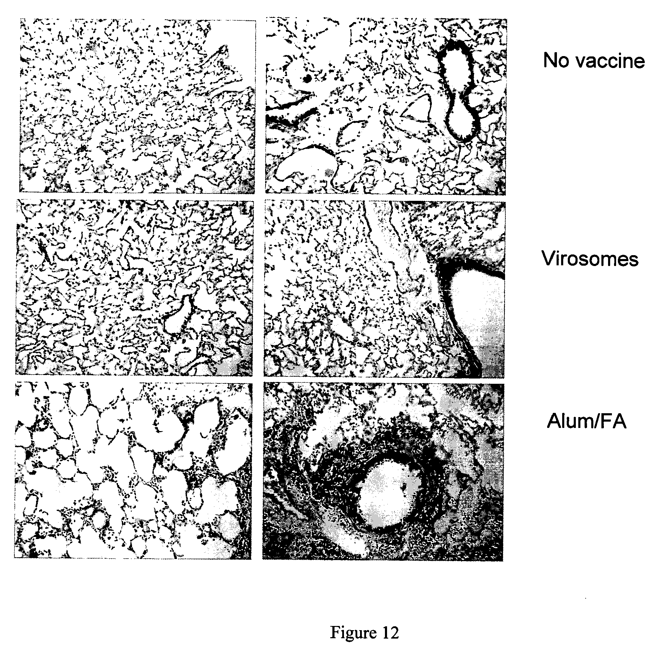

### Figure 12 Lungs of cotton rats after vaccination with RS virus/lipopeptide virosomes or aluminium-adjuvanted, formaldehyde inactivated virus.

Cotton rats were vaccinated with RS virus A2/lipopeptide virosomes (middle row) or aluminium-adjuvanted, formaldehyde inactivated virus (bottom row) as described, and challenged with live virus 14 days after the last vaccination. A control group was challenged but not vaccinated (top row) 5 days later the rats were euthanized, the lungs were fixed while infleted at a constant pressure, and Giemsa-stained slices were prepared. The left hand panels show the alveoli at high magnification, the right hand panels an overview at low magnification (the same magnification was used for each column). The widespread destruction of alveoli in the lungs of the third row, the interstitial eosinophiles in the alveoli, and the large infiltrates are obvious.

### Examples

### Example 1: Comparison of different methods for the formation of virosomes from RS Virus.

RS Virus, strain A2, acquired from the ATCC, was produced by growing the virus on Hep-2 or Vero cells, using methods known to persons skilled in the art, and then purified, preferably by differential or density gradient ultracentrifugation or a combination thereof, and subsequently inactivated by beta-propiolactone according to established standard procedures.

For solubilization by dicaproyl-phosphatidylcholine (DCPC) or octa-ethylene glycol mono-N-dodecylether (C12E8), RS virus (1.3 mg viral protein each) was pelleted by ultracentrifugation, and dissolved by repeated pipetting in 100 mM DCPC or 100 mM C12E8 in HNE buffer (145 mM NaCL, 5 mM, 1 mM EDTA, pH 7.4), followed by an incubation at 4-37°C for 30 min. The material was then pelleted by centrifugation for 40 min. at 40 000 rpm in a TLA 100.3 rotor of a table-top ultracentrifuge (Beckman), and the pellets, containing the viral RNA, were discarded. The supernatants were saved for reconstitution. A mixture of phospholipids and cholesterol, in a molar ratio of 2 µmol of phosphatidylcholine (purified from chicken eggs), per 1 µmol of phosphatidylethanolamine (transesterified from phosphatidylcholine, purified from chicken eggs), 3.5 µmol of sphingomyelin, and 3.5 µmol cholesterol, was prepared in a 5 ml solution of chloroform/methanol (2:1 molar ratio). All lipids were from Avanti Polar Lipids, Birmingham, Alabama, USA. 357 µl of this mixture were dried in vacuo, and both supernatants prepared as described above were added to the dry lipid films.

C12E8 was then removed from the supernatant lipid mix by shaking with 284 mg of Biobeads SM-2 (Bio-Rad) for one hour, followed by addition of a further 143 mg of Bio-Beads, and shaking for a further 10 min, essentially as described for the formation of influenza virosomes (Stegmann et al., 1987).

To the DCPC prepared lipid/supernatant mixture, 292 µl of the lipopeptide N-palmitoyl-S-2,3(bispalmitoyloxy)-propyl-cysteinyl-seryl-(lysil)₃-lysine, prepared as a 5 mg/ml solution of the lipopeptide, dissolved in HNE-buffer containing 100 mM DCPC, was further added (resulting in a ratio of 1mg of viral supernatant protein to 1 mg of lipopeptide). For the removal of DCPC and reconstitution of the membrane, the supernatant or supernatant mixtures. were transferred to a 10,000 MW cut-off dialysis cassette ('Slide-A-Lyzer', Pierce Chemical Company, Rockford, IL, U.S.A.) and dialyzed against 11 of HNE-buffer for 6 hr at 4 °C, and subsequently against 11 fresh HNE-buffer for 15 hr at 4 °C, followed by a further 2 hours against 11 fresh HNE-buffer for 15 hr at 4 °C.

The results of these experiments were analyzed by equilibrium density sucrose gradient analysis. A sample of the dialyzed material was loaded on a linear gradient of 10-60% (w/v) sucrose in HNE-buffer and centrifuged for at least 60 hrs at 100,000 x g at 4 °C (Figures 1 and 2). After centrifugation the gradient was fractionated, and the protein and phospholipid phosphate concentrations in the fractions were determined, and plotted vs. the fractions density as measured by refractometry. The results shown in Figure 1 clearly show that the C12E8 treated material was found as protein and lipid in all fractions, indicating that little or no virosomes were formed, whereas DCPC reconstituted material (Figure 2) showed a single peak on the gradient, at a density of about 1.1288 g/ml, indicating that virosomes were formed by this method. Thin-layer chromatography analysis (not shown) further revealed that the lipopeptide was also predominantly recovered in this peak. Similar results were obtained for virosomes not containing the lipopeptide. SDS-PAGE analysis (not shown) confirmed the presence of the RS virus membrane proteins, F and G in the virosomes. The formation of virosomes by the DCPC method was further confirmed by negative stain electron microscopy of the virosomes as shown in Figure 3. Virosomes were about 100 nm in diameter.

In conclusion, surprisingly, although applicant had earlier demonstrated that influenza virus membranes could be reconstituted by C12E8 but not by a series of other detergents, RS virosomes could not be formed by C12E8 detergent removal, but were readily formed by DCPC dialysis.

### Example 2: Production of reconstituted viral membranes comprising membrane material from both influenza and RS virus.

Influenza virus, strain A/Panama/2007/99, was produced by growing virus acquired from the World Influenza Center or the American Type Tissue Culture Collection (ATCC), using methods known to persons skilled in the art, for instance by growing the virus on embryonated eggs or cultured cells. The virus was then purified, preferably by differential or density gradient ultracentrifugation or a combination thereof, and subsequently inactivated by formaldehyde according to established standard procedures.

RS Virus, strain A2, acquired from the ATCC, was produced and inactivated as described in example 1.

For solubilization by DCPC, RS virus or influenza virus were first sedimented by ultracentrifugation, and subsequently resuspended with 0.7 ml HNE-buffer, containing 100 mM DCPC. After a 30 min incubation at 4°C, the viral membrane was solubilized, and the nucleocapsids were removed by centrifugation for 40 min at 100,000xg at 4 °C. The supernatants were mixed at a 1:1 ratio weight ratio of protein and used for reconstitution.

For the incorporation of lipopeptides, for example the lipopeptide N-palmitoyl-S-2,3(bispalmitoyloxy)-propyl-cysteinyl-seryl-(lysil)₃-lysine, a 1 mg/ml solution of the lipopeptide, dissolved in HNE-buffer containing 100 mM DCPC, was added to the supernatants, at a ratio of 1mg of viral proteins to 1 mg of lipopeptide.

For reconstitution, the supernatant mixture was dialyzed as in example 1 above. The formation of virosomes was confirmed by equilibrium density gradient analysis as described in example 1. A single peak consisting of protein, lipid and if added, lipopeptide was found near a density of 1.12 g/ml, indicating that virosomes were formed. The formation of virosomes was further analyzed by electron microscopy (Figure 4). The viral proteins can be seen to protrude form the virosomes as spikes. Influenza/RS virosomes were larger, about 200 nm in diameter, than RS virosomes (about 100 nm). Influenza spikes are much more prominent than those of paramyxoviruses such as RS virus. Similar results were obtained with different strains of RS virus. In conclusion, mixed reconstituted viral membranes comprising material from both influenza and RS virus could be formed by the DCPC method.

### Example 3: Immunization experiments using DCPC-reconstituted viral membranes from RS virus alone or from RS virus/influenza mixed membranes with or without the lipopeptide N-palmitoyl-S-2,3(bispalmitoyloxy)-propyl-cysteinyl-seryl-(lysil)₃-lysine.

Vaccination experiments involving intramuscular application of reconstituted viral membranes, prepared as described in examples 1 and 2, comprising either the membrane proteins and lipids of both RS virus and influenza virus or RS virus alone, with or without lipopeptides, were carried out in Balb/C mice. The animals were immunized by intramuscular injection of antigen comprising 5 µg of viral protein on days 0 and 14. Blood samples were taken on day 0, 14, and 35. Samples were analyzed for RS virus-specific IgG by ELISA, using ELISA plates coated with beta-propiolactone-inactivated RS virus. The results, shown in Figure 4, clearly indicate that anti-RS virus antibodies were induced by all the vaccines, about an order of magnitude more antibody was produced 14 days after the first or second injection of RS virosomes comprising lipopeptides, than RS virosomes without lipopeptides. Moreover, surprisingly in the light of the literature, and earlier finding by applicant showing that influenza virus is an excellent carrier for the delivery of antigens to the immune system, although antibody titers induced by mixed influenza/RS virus mixed virosomes were also boosted if lipopeptide was present in the membrane, titers achieved by influenza/RS virus with lipopeptide were only about similar to those induce by RS virosomes alone with lipopeptide (Figure 5).

RS proteins were prepared for SDS-PAGE electrophoresis, with and without reduction by dithiotreitol (20 mM), separated by electrophoresis, and transferred onto nitrocellulose membranes by methods known in the art. Sera from the mice in each group were pooled and equal aliquots used for Western Blot analysis of viral proteins. As shown in Figure 6, all mouse sera recognized both the F and the G viral membrane proteins of RS virus to some extent, but lipopeptide-adjuvanted virosomes induced higher antibody concentrations, consistent with the ELISA results, and clearly recognized the F1 subunit of the F protein. The highest MW protein shown in non-reduced samples is probably a multimer of F, which is not sufficiently denatured under these conditions.

### Example 4: Lung histopathology following live virus challenge after vaccination with RS virus vs. RS virus/influenza mixed, lipopeptide adjuvanted virosomes.

Balb/C mice are, as human neonates, inherently biased to a Th2 response, and are therefore a critical model of RS virus vaccine safety. Virosomes comprising the lipopeptide *N-*palmitoyl-S-2,3(bispalmitoyloxy)-propyl-cysteinyl-seryl-(lysil)₃-lysine were produced from RS virus, strain A2 alone or RS virus A2/influenza as described above, at a 1:1 weight ratio of RS virus to influenza virus membrane protein for the mixed virosomes, and a 1:1 weight ratio of lipopeptide to viral protein, and Balb/C mice were immunized twice by intramuscular injection of 5 µg of viral protein with a 14-day interval. 14 days after the last injection, mice were challenged with approximately 10⁶ TCID50 of live RS virus A2 virus. Four days after challenge, mice were euthanized, their lungs were fixed with 4% saline formaldehyde and stained using Giemsa stain. Mice vaccinated with RS virosomes had intact alveoli, showed no signs of eosinophilia or alveolitis, and some small lymphocyte infiltrates limited to one side of a blood vessel (Figure 7). In contrast, mice vaccinated with mixed RS virus/influenza virosomes showed large lymphocyte infiltrates, that were sometimes seen to constrain blood vessels or airducts (Figure 8).

In conclusion, although adjuvanted preparations of inactivated RS virus material, such as whole virus, are capable of inducing enhanced disease, virosomes comprising the reconstituted RS virus membrane demonstrate a favorable safety profile.

### Example 5: Th1/Th2 response after a live virus challenge, following vaccination with RS virosomes, comprising lipopeptide adjuvant, in inherently Th2 biased Balb/C mice.

Several groups of six Balb/C mice were immunized twice with 5 µg of RS virus A2 virosomes comprising lipopeptide as described in example 4, and challenged with 10⁶ TCID50 live virus 14 days after the last injection. A blood sample was taken at the time of challenge and at euthanasia, 4 or 7 days later. RS virus-specific serum IgG1 and IgG2a antibodies were determined using ELISA plates, coated with beta-propiolactone inactivated RS virus, and calibrated against purified mouse IgG1 or IgG2a (From Bethyl Laboratories, Bethesda, MD, USA). No RS virus-specific antibodies were found at any of these time points in control mice that were challenged with live virus without prior vaccination. The results, shown in Table 3, clearly indicate a mixed type Th1/Th2 response, both before and after challenge, in mice that are heavily Th2 biased, confirming the safety of this type of vaccine, confirming the conclusions of example 5.

**Table 3: IgG1 and IgG2a after vaccination and before or after challenge of RS virus/lipopeptide vaccinated mice**

| | **IgG1 (µg/ml)** | **IgG2a (µg/ml)** |
|---|---|---|
| Before challenge | 10.6 ± 3,26 | 4,83 ± 2,70 |
| 4 days after challenge | 11.0 ± 4,00 | 14.5 ± 9,68 |
| 7 days after challenge | 14.3 ± 6,82 | 8.17± 5,88 |

### Example 6: Induction of MHC-I and MHC-II restricted IFNγ production by using spleen cells from mice vaccinated with RS virosomes and challenged with live virus.

8-12 week old Balb/C mice were immunized twice with 5 µg of RS virus A2 virosomes comprising lipopeptide as described in example 4, and challenged with 10⁶ TCID50 live virus 14 days after the last injection. The mice were euthanized and their spleen cells collected. The spleen cells were plated onto 96-well plates coated with anti-mouse IFNγ antibodies (rat IgG1, from BD Pharmingen, San Diego, CA, USA), and cultivated overnight. The cells were than either stimulated with beta-propiolactone inactivated RS virus to induce predominantly an MHC-II restricted, or with RS virus-infected, lethally irradiated P 815 cells (infected with RS virus strain A2 at an m.o.i. of 0.1 for 16 hours at 37°C and irradiated with 100 Gy for 10 min) to induce predominantly an MHC-I response, and incubated 48h at 37°C. The medium was then aspirated and the cells were lysed with H2O, washed with phosphate-buffered saline (PBS)/Tween, and then 0.0625 µg per well of rat anti-mouse IFN-γ coupled to biotin (Pharmingen San Diego, CA, USA), incubated for one hour at 37°C, washed with PBS/Tween, and incubated with streptavidin-alkaline phosphatase (Pharmingen), after which agarose-containing substrate solution containing 5-bromo-4-chloro-3-indolylphosphate in water was added at 45°C, and the plates were kept in the refrigerator until spots could be counted. The number of spots in wells containing equal numbers of non-stimulated cells was subtracted from the numbers counted in wells.

The results are shown in Figure 9. Vaccination of the mice with the virosome vaccine clearly led to a rapid induction of significant IFNγ production, a desirable aspect of an RS virus vaccine.

### Example 7: Protection against live virus challenge after vaccination

8-12 week old Balb/C mice were immunized twice with either 5 µg of RS virus A2 or 5 µg of an RS virus B strain virosomes comprising lipopeptide as described in example 4, and challenged with 10⁶ TCID50 live virus of the respective strain 14 days after the last injection. As a control, non-vaccinated mice were challenged with the same quantity of virus. The mice were euthanized, their lungs collected, and ground on ice using a polytron, centrifuged for 10 min at 800 rpm to remove tissue, and then virus titers in the supernatant were determined on Hep-2 cells, after an incubation for six days at 37°. The results, shown in Figure 10 clearly show that more than a three ¹⁰log reduction in titer was induced by the virosome vaccine, in fact producing sterilizing immunity.

### Example 8: Protection against live virus challenge after virosomal vaccination at different ratios of viral protein to lipopeptide

8-12 week old Balb/C mice were immunized twice with 5 µg of RS virus A2 virosomes comprising lipopeptide at 1:1, 1:2, or 0:1 weight ratio of lipopeptide to viral protein, respectively, and challenged with 10⁶ TCID50 live RS virus A2 14 days after the last injection. As a control, non-vaccinated mice were challenged with the same quantity of virus. Virus titers in lung tissue were determined as in example 7. The results, shown in Figure 11 clearly show that sterilizing immunity was induced by all versions of the virosome vaccine.

### Example 9: Virosome vaccine safety in cotton rats after live challenge; comparison with formaldehyde-inactivated, aluminum-precipitated, RS virus vaccines.

6-8 week old cotton rats (Sigmodon Hispidus) were immunized twice intramuscularly, with an interval of two weeks, either with 10 µg of RS virus A2 virosomes comprising the lipopeptide *N*-palmitoyl-S-2,3(bispalmitoyloxy)-propyl-cysteinyl-seryl-(lysil)₃-lysine, at a 1:1 weight ratio of lipopeptide to viral protein, or with 50 µl of a mock-up version of "Lot 100"vaccine . The latter was produced by infecting Hep-2 cells with RS virus-A2 at a multiplicity of infection of 0.1 and incubing for 3 days in fetal calf serum-containing tissue culture medium, and then the cell culture supernatant was harvested, clarified by centrifugation at 2000 rpm for 10 min., after which the supernatant was treated with formalin (0.025% final concentration) for 3 days at 37°C. Virus was concentrated 100-fold from this solution by ultracentrifugation, then mixed overnight with 0.5 ml of aluminum hydroxide (Gerbu, Alhydrogel®) per 4 ml, centrifuged at 1000 rpm, and then the pellet was taken up into 0.5 ml of HNE buffer. Seven weeks after the last vaccination, animals were challenged with 2.2 * 10⁶ TCID₅₀ of live virus. Five days after the challenge, the animals were euthanized, their lungs fixed with 4% formaldehyde/saline overnight at a constant pressure of 20 cm water column, to keep the lungs inflated, and lung slices were stained with Giemsa and examined by microcopy (Figure 12). As a control, non-vaccinated cotton rats were also challenged with live virus. As shown in Figure 12, non vaccinated infected control animals showed intact alveoli with no signs of alveolitis, with some mast cells present and no lymphocyte or eosinophile infiltrates 5 days after infection. Virosome-vaccinated rats showed intact alveoli, some lymphocyte infiltration, and the presence of mast cells and scattered alveoli. On the other hand, vaccinated with aluminum-adjuvanted, formaldehyde-inactivated virus showed extensive sign of alveolitis with widespread destruction of alveoli, interstitial infiltration by eosinophiles, and very large infiltrates containing eosinophiles and lymphocytes surrounding all airways.

In conclusion, lipopeptide-adjuvanted RS virosomes are a safe and effective vaccine for RS virus.

### References

Almeida, J.D. et al. (1975) The Lancet 699-701
Arkema, A. (2000) Vaccine 18: 1327-1333
Bungener, L (2002) Vaccine 20: 323-338
Cusi, M.G. et al. (2002) Vaccine 20, 3436-3442
De Jonge, J. et al. (2006) Biochim Biophys Acta. 1758,527-36.
Dudas, R.A., and Karron, R.A. (1998) Clin. Microbiol. Rev. 11, 430-439
Feldman, S.A. et al. (2001) Arch. Virol. 146:2369-2382
Lamb, R.A. et al. (2006) Virology 344, 30-37
Mutsch et al. (2004) N. Engl. J. Med. 26:896-903
Openshaw, P.J.M. (2002) Respir. Res. 2002 3(suppl):S15-S20
Remington's Pharmaceutical Science (1980) 15th ed., Mack Publishing, Easton, PA
Stegmann, T. et al. (1987) EMBO Journal 6, 2651-2659
Tausk, R. J. M. et al. (1974) Biophysical Chemistry 1, 175-183

## Claims

1. A reconstituted viral membrane, comprising membrane proteins and natural lipids of a Respiratory Syncytial virus.

2. A mixed reconstituted viral membrane, comprising the reconstituted viral membrane according to claim 1, and a reconstituted membrane of another enveloped virus, comprising membrane proteins and natural lipids of said other enveloped virus.

3. The mixed reconstituted viral membrane according to claim 2, wherein said other enveloped virus is influenza.

4. The (mixed) reconstituted viral membrane according to any one of claims 1 to 3, further comprising an amphiphilic adjuvant.

5. The (mixed) reconstituted viral membrane according to claim 4, wherein the amphiphilic adjuvant is a lipopeptide, a glycolipid or a peptide.

6. The (mixed) reconstituted viral membrane according to any one of claims 4 or 5, wherein the amphiphilic adjuvant is a ligand for a mammalian Toll-like receptor.

7. The (mixed) reconstituted viral membrane according to claim 5, wherein the lipopeptide is selected from the group consisting of: N-palmitoyl-S-2,3(bispalmitoyloxy)-propyl-cysteinyl-seryl-serine, S-2,3(bispalmitoyloxy)-propyl-cysteinyl-seryl-serine, *N*-palmitoyl-S-2,3(bispalmitoyloxy)-propyl-cysteinyl-seryl-(lysil)₃-lysine, S-2,3(bispalmitoyloxy)-propyl-cysteinyl-seryl-(lysil)₃ -lysine, *N-*palmitoyl-S-2,3(bisoleoyloxy)-propyl-cysteinyl-seryl-(lysil)3-lysine, S-2,3(bisoleoyloxy)-propyl-cysteinyl-seryl-serine-(lysil)₃ -lysine, *N*-palmitoyl-S-2,3(bismyristoyloxy)-propyl-cysteinyl-seryl-(lysil)₃-lysine, S-2,3(bismyristoyloxy)-propyl-cysteinyl-seryl-(lysil)₃ -lysine, *N*-palmitoyl-S-3(palmitoyloxy)-propyl-cysteinyl-seryl-(lysil)₃ -lysine and *N*-palmitoyl-S-2,3 hydroxy-propyl-cysteinyl-seryl-(lysil)₃-lysine, *N*-palmitoyl-S-2,3(bispalmitoyloxy)-propyl-cysteinyl-seryl-(prolyl)₃-proline, *N*-palmitoyl-S-2,3(bispalmitoyloxy)-propyl-cysteinyl-seryl-(glutaminyl)₃-glutaminic acid.

8. The (mixed) reconstituted viral membrane according to claim 5, wherein the glycolipid is a phosphatidyl inositol mannoside, an alpha-galactosylceramide, or a modified lipopolysaccharide having reduced toxicity.

9. A virosome comprising the (mixed) reconstituted viral membrane according to any of claims 1 to 8.

10. A method of producing a reconstituted viral membrane according to any one of claims 1 and 4-8 or a virosome according to claim 9, comprising the steps of:
(a) contacting an enveloped virus with a solution comprising a short-chain phospholipid allowing solubilization of the viral envelope of said virus
(b) optionally, removing the nucleocapsid of the solubilized virus by centrifugation
(c) optionally, adding an amphiphilic adjuvant to the solubilized viral envelope
(d) optionally, adding lipid(s) to the solubilized viral envelope
(e) removing the short-chain phospholipid from said solution by dialysis or filtration allowing formation of a reconstituted viral envelope.

11. A method of producing a mixed reconstituted viral membrane according to any one of claims 2 to 8 or a virosome according to claim 9, comprising the steps of:
(a) contacting a first enveloped virus with a solution comprising a short-chain phospholipid allowing solubilization of the viral envelope of said virus
(b) contacting a second enveloped virus with a solution comprising a short-chain phospholipid allowing solubilization of the viral envelope of said virus
(c) optionally, removing the nucleocapsid from both viruses from the solubilized virus by centrifugation
(c) mixing the solubilized membranes of both viruses
(d) optionally, adding an amphiphilic adjuvant to the mixture
(e) optionally, adding lipid(s) to the mixture
(f) removal of the short-chain phospholipid from said mixture by dialysis or filtration allowing formation of a mixed reconstituted viral envelope comprising lipids and proteins of both viruses.

12. The method according to claims 10 or 11 wherein said phospholipid has acyl chains with fewer than 9 nine carbon atoms.

13. The method according to claims 10 to 12 wherein the phospholipid is phosphatidic acid, phosphatidylethanolamine, phosphatidylserine or phosphatidylcholine.

14. The method according to anyone of claims 10 to 13 wherein said phospholipid has a critical micelle concentration (cmc) of larger than 0.1 mM

15. The method according to anyone of claims 10 to 14 wherein said phospholipid comprises 1,2-diheptanoyl-*sn*-phosphatidylcholine or 1,2-dicaproyl-*sn-*phosphatidylcholine.

16. Use of a (mixed) reconstituted viral membrane according to any one of claims 1 to 8 or of a virosome according to claim 9 or obtained by a method according to any one of claims 10 to 15, for the preparation of a vaccine.

17. Use of a (mixed) reconstituted viral membrane according to any one of claims 1 to 8 or of a virosome according to claim 9 or obtained by a method according to any one of claims 10 to 15, for the preparation of a vaccine for the prevention, alleviation or treatment of a RSV infection.

18. A pharmaceutical composition comprising a (mixed) reconstituted viral membrane as defined in any one of claims 1 to 8, or a virosome as defined in claim 9, or obtained by a method according to any one of claims 10 to 15, and optionally a pharmaceutically acceptable carrier.

19. The pharmaceutical composition according to claim 18, whereby the composition is suitable for intranasal, oral or parenteral administration.

20. A vaccine comprising a (mixed) reconstituted viral membrane of any one of claims 1 to 8, or a virosome of claim 9 or obtained by a method according to any one of claims 10 to 15 and, optionally, a pharmaceutically acceptable carrier or diluent.

21. The vaccine according to claim 20, suitable for intranasal, intravaginal, oral or parenteral administration.

22. A method for the vaccination of a subject comprising the step of administering a (mixed) reconstituted viral membrane according to any one of claims 1 to 8 or of a virosome according to claim 9 or obtained by a method according to any one of claims 10 to 15.

23. A method for the vaccination of a subject for the prevention, alleviation or treatment of a RSV infection comprising the step of administering a reconstituted viral membrane according to any one of claims 1 to 8 or of a virosome according to claim 9 or obtained by a method according to any one of claims 10 to 15, optionally in combination with a pharmaceutically acceptable carrier or diluent and/or an adjuvant.
